# EUROPEAN PATENT SPECIFICATION

(11) **EP 1 494 585 B1**
(45) Date of publication and mention of the grant of the patent: **27.05.2009**
(21) Application number: 03745363.6
(22) Date of filing: 28.03.2003
(51) Int. Cl.: A61B 5/08, A61M 16/08

(54) **VENTILATION APPARATUS FOR PULMONARY SCINTIGRAPHY**
BEATMUNGSGERÄT FÜR DIE PULMONALE SZINTIGRAPHIE
APPAREIL DE VENTILATION POUR SCINTIGRAPHIE PULMONAIRE

(30) Priority: 28.03.2002 IT PI20020018
(43) Date of publication of application: 12.01.2005
(73) Proprietor: Azienda Ospedaliera Pisana, 56127 Pisa PI (IT)
(72) Inventor: ALBERTELLI, Roberto, I-56018 Tirrenia (IT)
(74) Representative: Celestino, Marco
(86) International application number: PCT/IB2003/001156
(87) International publication number: WO 2003/082109

(56) References cited:
- EP-A- 0 119 864
- EP-A- 0 262 239
- EP-A- 0 911 050
- US-A- 3 666 955
- US-A- 4 803 977

## Description

The present invention relates to the medical field and in particular it relates to a ventilation apparatus for pulmonary scintigraphy, capable to provide a uniform distribution of a marking substance in a patient's lungs.

Furthermore, the invention relates to a method for inhalation of this substance.

### Background.of the invention

As known, Lungs Ventilatory Scintigraphy is a technique for detecting the distribution of lungs ventilation in patients with pathology of the respiratory system such as bronchial asthma, chronic bronchitis and emphysema. It consists in ventilating into the patient an inert gas such as ¹³³Xe, ¹²⁷Xe, the ⁸¹mKr, or aerosol, usually ⁹⁹mTc-DTPA (diethylen-triamino-pentacetic acid) or sulphide colloidal ⁹⁹mTc, for then looking at its distribution in the lungs.

With the perfusion and ventilation systems presently in use, the patient breathes in marked aerosol, i.e. aerosol with dissolved radioactive material, through a mouthpiece connected on an end of a tube and connected at the other end to an aerosol apparatus, and breathes out the surplus marker through the same tube towards a radioactive material collecting filter. The air pressure is maintained steady when breathing in and out, so that the marker is deposited in the largest bronchial branches of the respiratory system. The radioactive material, therefore, deposits especially in the main bronchial branches, colliding against their walls, whereas it reaches the peripheral branches much slowly.

For taking a full picture with a "gamma camera" of the main and peripheral respiratory tubes the patient must ventilate them with a traditional aerosol apparatus for about 20/30 minutes. In fact, only such a long ventilation allows the radioactive substances to deposit also in the most peripheral tubes, in order to take an overall picture of the lungs.

On the other hand, for diffusing the radioactive material in the lungs, enough to provide readable distribution images of the ventilation (at least 1Kc/sec), not only the above aerosol time is necessary (about 20/30 minutes) but also time is necessary for taking the images twice, i.e. preliminary images, immediately after counting 1KC/sec, and deferred images, three/four hours later. This double test, in two different times, is necessary to avoid that the second image shows a radioactive accumulation in the main respiratory tubes. Such accumulation of radioactive material affects the acquisition of the first image, which since they are "unclean" cannot be read, unless comparing it with the second, for ascertaining possible pulmonary occlusions. In order to obtain "a clean image" it is necessary to wait for about four hours, so that the mucociliary system eliminates partially the accumulation of radioactive material. Notwithstanding the deferred images do not show said radioactive accumulation, however they cannot avoid to show the marker swallowed with the saliva through the oesophagus and the stomach, since scintigraphy detects it.

Another problem is that a scintigraphy test, during Non-Invasive Mechanical Ventilation with nasal mask, cannot be carried out without affecting the pressure control on which the apparatus is based and without polluting the apparatus and the environment with the exhaled marked aerosol.

In US 4803977 an apparatus for radiology, capable of supplying a measured amount of radioactive mist or marker to a patient, using a three way collector with one-way valves for breathing-in and breathing-out paths.

In EP 0262239 a respiratory device is described for monitoring inhalation and exhalation and not to radiology, having a connection three way pipe with an exhalation resistance arranged in the exhalation path and a one way valve in the inhalation path.

In US 3666955 an automatic control system is described for radioactive studies on the lung ventilation is provided, having a mouth collector with electrovalves.

### Summary of the invention

It is therefore object of the present invention to provide a ventilation apparatus for pulmonary scintigraphy that provides a uniform distribution of the marked aerosol in the bronchial branches, i.e. without hyper accumulation of marker, reducing the impact on the bronchial branches and making easier a direct peripheral distribution of the marker.

It is another object of the present invention to provide a ventilation apparatus for carrying out pulmonary scintigraphy even on not cooperating patients.

It is also object of the present invention to provide a ventilation apparatus for pulmonary scintigraphy capable of reducing the time necessary for executing a scintigraphical test.

It is a further object of the present invention to provide a ventilation apparatus for pulmonary scintigraphy for carrying out at the same time a non invasive mechanical ventilation with nasal mask, without affecting the pressure cycles on which the apparatus works and without polluting the apparatus and the environment with exhaled marked aerosol.

These and other objects are achieved by the ventilation apparatus for pulmonary scintigraphy, according to the present invention, comprising:
- a feeding duct defining a flow of marked aerosol;
- an outlet duct for exhaled or surplus aerosol;
- an inhalation collector where said feeding and outlet ducts converge; and
- a one-way interposition element movable between a first position, where it allows the flow of marked aerosol into said inhalation collector when breathing in, and a second position, where it stops or limits the flow into said inhalation collector of marked aerosol when breathing out.

The inhalation collector is located near the zone of inhalation, either through nose or mouth, to assure that the inlet marked aerosol flows through a way different from the outlet marked aerosol.

Said one-way interposition element has preferably intermediate positions in addition to an open and a closed position, in order to follow the breathing demand of the patient. In fact, when breathing in and out a gradient of pressure is produced in the inhalation collector suitable for creating a flow of marked aerosol following the physiological respiration of the patient.

Advantageously, the one-way interposition element is a flap valve.

In particular, the one-way interposition Element is a flap valve formed by a stiff plastic foil that is elastically flexible.

The foil deflects only in one direction owing to an annular abutment, added to or made in the collector or in the tube, on which the foil same is connected at a side and on which it rests when in closed position. Then, each foil has a fixed portion, integral to the inner collector wall, about which the other portion of the foil can rotate between the first and the second position. The annular abutment is co-axial to the portion of the collector, or of the duct in which it is arranged, and has inner diameter smaller than that of the respective foil.

Advantageously, the flow of marked aerosol coming from a nebulizer is led into the air feeding duct upstream of the collector.

Alternatively, the flow of marked aerosol coming from a nebulizer is led directly into the collector through a duct different from that of the air feeding duct.

In a first embodiment of the invention, the inhalation collector is a three-way collector, with a central chamber and three apertures. In particular, the following are provided:
- a first opening for connecting said central chamber with a feeding duct of marked aerosol;
- a second opening for connecting the central chamber with an outlet duct of surplus marker connected to a radioactive material collecting filter;
- a third opening for connecting the central chamber with an element of inhalation through nose or mouth;
- a first flap one-way valve at the first opening for an inlet flow;
- a second flap one-way valve at the second opening for an outlet flow.

More in detail, when the patient breathes in the marked aerosol the flap valve at the first opening opens completely, whereas the valve at the second opening is completely closed. When breathing out, instead, the first valve blocks partially or completely the relative aperture responsive to local pressures, reducing the flow of the aerosol, already at a low level in a bi-level ventilation, whereas the second valve opens and allows to dispose of the exhaled flow, thus following the patient's respiration.

In other words, the first valve reduces the inlet flow and allows it completely when breathing in. This creates a vortex in the large bronchial branches, without allowing the radioactive material to deposit by a stirring action. When breathing out, the surplus radioactive material is eliminated, leaving in the lungs only that necessary for marking. This way, the bronchial branches can be marked in a few minutes (from about 2 to 6 minuted) up to the final marking, and in this time it is possible to take the radiographs for looking at the transient phases .

In a second embodiment of the invention, the inhalation collector is a four-way collector, with a central chamber and four apertures. In particular, the following are provided:
- a first opening for connecting said central chamber with a feeding duct of marked aerosol;
- a second opening for connecting the central chamber with an outlet duct of surplus marker connected to a radioactive material collecting filter;
- a third opening for connecting the central chamber with a feeding duct of air for non invasive mechanical ventilation;
- a fourth opening for connecting the central chamber with an element of inhalation through nose or mouth;
- a flap one-way valve at the first opening for the inlet aerosol flow.

### Brief description of the drawings

Further characteristics and advantages of the present invention, will be made clearer with the following description of possible embodiments, exemplifying but not limitative, with reference to the attached drawings, wherein:
- figure 1 shows diagrammatically a partially cross sectioned elevational side view of a first embodiment of a mouth ventilation apparatus for pulmonary scintigraphy;
- figure 1A is a enlarged view of a detail of figure 1 for showing the mechanism of operation of a possible embodiment of a one-way interposition element;
- figure 2 shows diagrammatically a perspective view of the apparatus of figure 1 applied to a patient;
- figure 3 shows a perspective view of a first embodiment of an inhalation collector used in the apparatus of figure 1;
- figure 4 shows the collector of figure 3 in a cross sectional view that shows the relative positions of the different interposition elements when breathing in;
- figure 4A shows diagrammatically the pressure trend versus time in the apparatus of figure 1 when breathing in;
- figure 5 shows the collector of figure 3 in a cross sectional view that shows the relative positions of the different interposition elements when breathing out;
- figure 5A shows diagrammatically the pressure trend versus time in apparatus of figure 1 when breathing out;
- figure 6 shows diagrammatically the pressure trend versus time in apparatus of figure 1 during a full cycle of breathing in and out;
- figure 7 shows diagrammatically a partially cross sectioned elevational front view of a ventilation apparatus for pulmonary scintigraphy according to an embodiment alternative to that of figure 1 with nasal ventilation;
- figure 8 shows a perspective view of the inhalation collector used in the apparatus of figure 7;
- figure 9 shows diagrammatically a perspective view of the location of different components in the apparatus of figure 7, relatively to a patient when breathing in.

### Description of a preferred embodiment

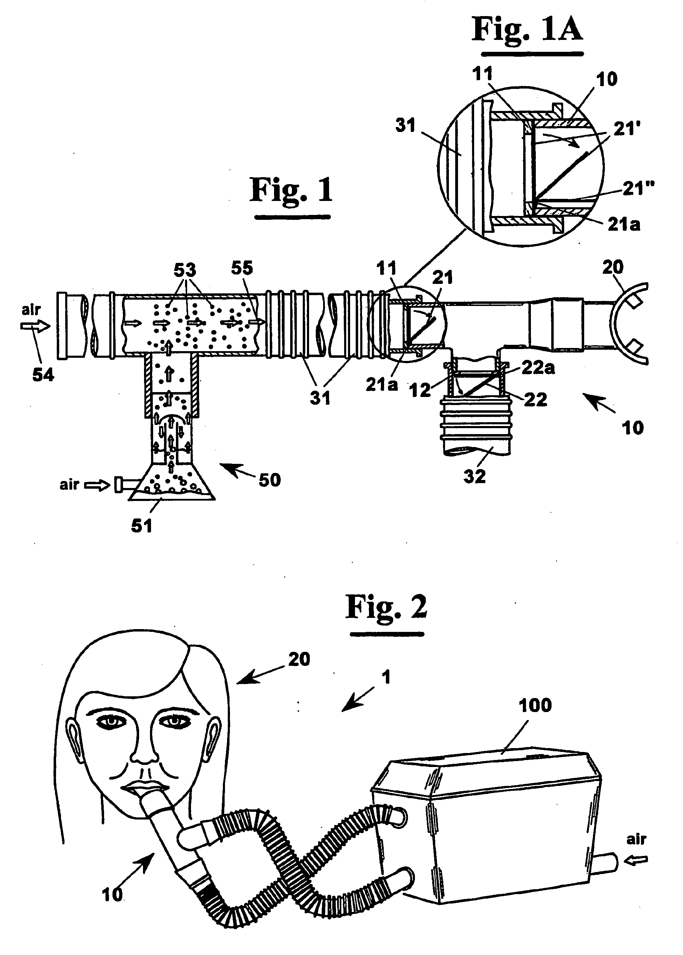

In figure 1 a first embodiment is shown of a ventilation apparatus 1 for pulmonary scintigraphy, according to the present invention.

It comprises an inhalation collector 10 to which an element of inhalation, for example a mouthpiece 20, a feeding duct 31, defining a flow of marked aerosol 55, and an outlet duct 32, for disposal of exhaled flow and any aerosol surplus, are connected. Normally the feeding air is of bi-level type, whereby the air flow rate when breathing out is low, even if always positive.

In particular, as shown in figure 1, the flow of marked aerosol 55 is obtained mixing upstream of collector 10 a current of nano-colloidal particles 53, marked for example with Tc-99m and delivered by a nebulizer 50, with an air flow 54.

As shown in figure 1 and in detail in figures 1A, collector 10 has, at the connections with ducts 31 and 32, one-way interposition elements 21 and 22. In particular, a first interposition element 21 is located at the connection with air feeding tube 31 and only allows inlet flow towards the patient, whereas a second interposition element 22 is located at the connection with outlet duct 32.

In the case shown in figures from 1 to 5, each one-way interposition element 21, or 22, is a flap valve formed by a foil of stiff thin plastic material capable of bending elastically associated to a ring abutment 11, or 12, solid or added, co-axial to the respective portion of collector 10 and having inner diameter smaller than the width of the respective valve. Each foil of plastic material 21 has a fixed portion 21a, integral to the inner wall of collector, 10, about which the other portion of foil 21 can rotate between a first position 21', where it stops or limits the flow 55 of marked aerosol, and a position 21", wherein a maximum cross section is offered to the flow of marked aerosol towards collector 10 (figure 1A). Valve 22 is made in a similar way for a flow exiting from collector 10. In the closed position elements 21 and 22 rest on rings 11 and 12.

The presence of the one-way valves 21 and 22 allows to breath the marked aerosol in the bronchial branches of the patient 20 in a way completely natural following the physiologic respiration and avoiding hyper-accumulation of marker in the largest bronchial branches.

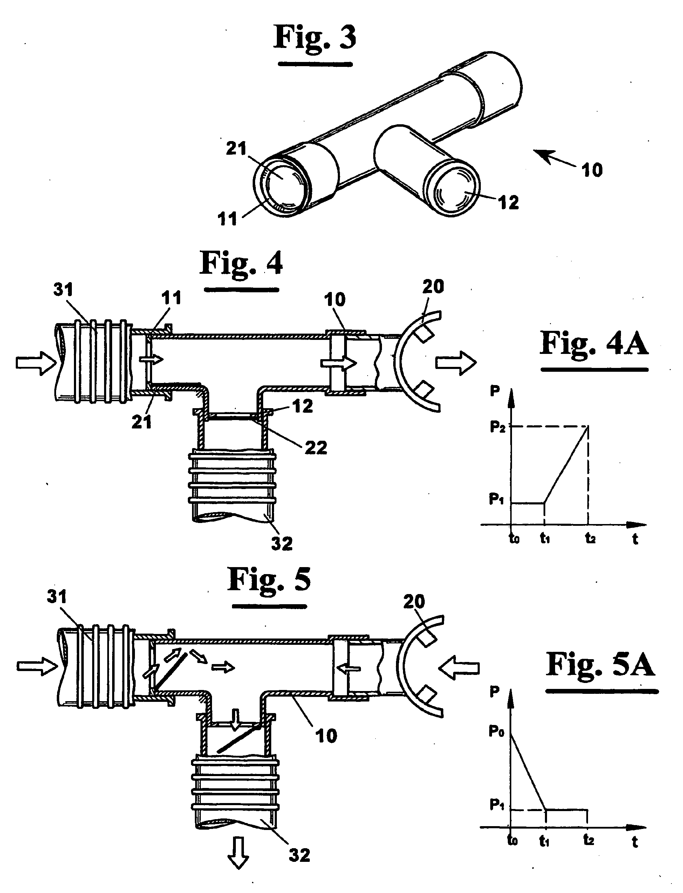

The device can be used, for example, as shown in figure 2, with respective ducts 31, 32 and mouthpiece 20 connected to collector 10 of figure 3. When breathing in, the pressure of the aerosol is maximum and the valve 21 is completely open (figure 4).

When breathing out, on valve 21 a certain pressure is made by the air exhaled by patient 20 in a direction opposite to the even low positive pressure of the fed aerosol flow, but of lower intensity. This causes valve 21 to move to an intermediate position that reduces but does not stop the aerosol flow into collector 10. Therefor, in collector 10 the flow of exhaled air and the surplus aerosol flow sum to each other increasing the pressure inside. This causes valve 22 to open for disposing of the surplus flow exhaled by patient 20 (figure 5). Valve 21, that reduces the aerosol flow towards collector 10 when breathing out and leaves it free when breathing in, when opens again produces a pressure variation that creates a vortex in the large bronchial branches of the patient without allowing the radioactive material 53 to deposit, owing to a stirring action of the vortex. When breathing out the radioactive material is eliminated along with the surplus air. This way the bronchial branches can be marked in a few minutes (from about 2 to 6 minutes).

What above said is graphically shown in figures 4A, 5A and 6 where qualitative trends of pressure (P) versus time (t) are indicated respectively when breathing in, when breathing out and during a full cycle of breathing in and out. In particular, when breathing in the pressure rises from a starting value P₁ up a maximum value P₂, whereas when breathing out the pressure decreases from a starting value P₀ to a value P₁<P₀, enough to keep valve 21 half open.

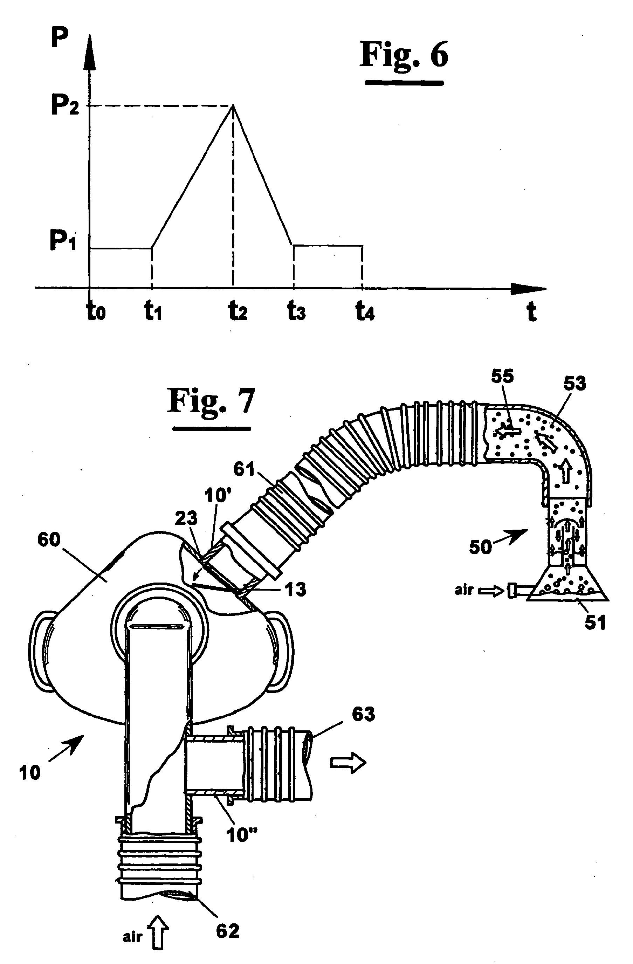

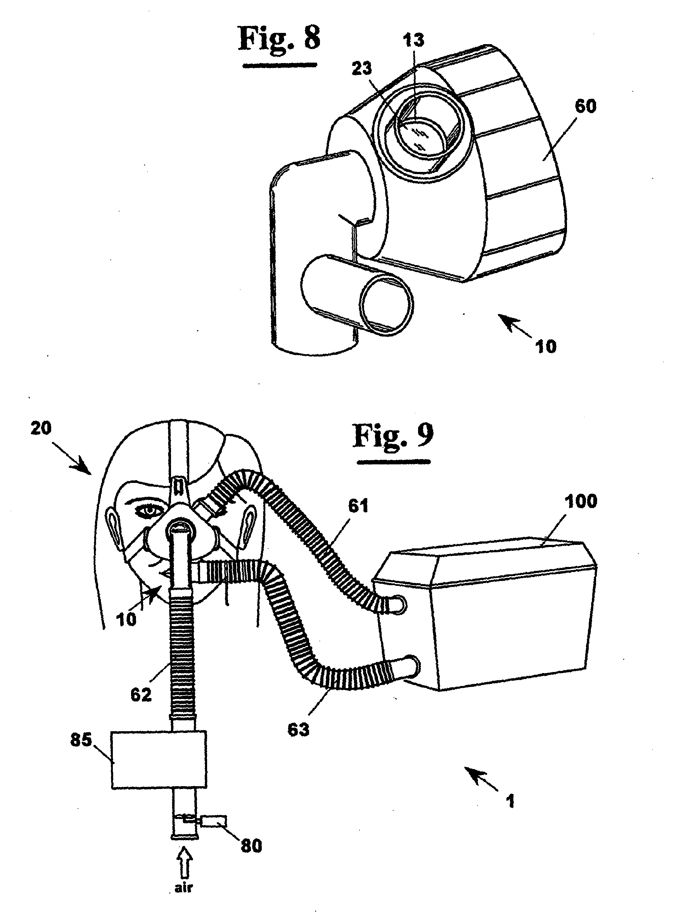

In figures from 7 to 9 an alternative embodiment is shown of a pulmonary ventilation scintigraphy apparatus alternative to that shown in figures from 1 to 5. This embodiment has the advantage of carrying out a pulmonary scintigraphy during a Non Invasive Mechanical Ventilation, or NIMV, without affecting the pressure cycles on which apparatus 1 works and without polluting apparatus 1 and the environment with exhaled marked aerosol. As shown in figure 7, a collector 10 integrated in a nasal mask 60 of an apparatus for mechanical ventilation is normally connected to a primer 80 (figure 9) that produces a flow of air towards the mask same.

For carrying out a pulmonary scintigraphy, using a similar apparatus, in nasal mask 60 a one-way valve 23 is arranged allowing only an inlet flow, like that above described (figure 1A). Valve 23 is located in collector 10 at a connection 10' with a feeding duct 61 of an aerosol flow 55 produced by a nebulizer 50. Nasal mask 60 is, furthermore, connected by a connection 10" of collector 10 to an outlet duct 63 of a exhalation flow containing the surplus marked aerosol, connected at the other end to a collecting filter, not shown, inserted into a lead walled box 100, in order to prevent scattering of radioactive material in the environment and to protect the operator.

During NIMV, at the moment of executing the test, it is sufficient to connect the feeding tube 61 to collector 10. In particular, nebulizer 50 works with a pressure sufficient to open valve 23 only when in the mask a vacuum is created caused by the inspiration of the patient, whereas it stops when breathing out. Differently from the previous case, the system mixes the air supplied by primer 80 and aerosol flow 55 of nano-colloidal particles 53 marked with Tc-99m coming from nebulizer 50 only in collector 10 and not before; therefore, the flow of marked aerosol to the patient 20 is adjusted by primer 80. This way, it is possible to measure the distribution of air supplied by primer 80 in the lungs of patient 20 thus allowing, with the aid of an imaging system, to check in real time the lungs apex-base ratio U/L.

## Claims

1. Ventilation apparatus (1) for pulmonary scintigraphy comprising:
- a feeding duct (31) defining an inlet flow of marked aerosol (55);
- an outlet duct (32) for exhaled / surplus outlet aerosol;
- an inhalation collector (10) where said feeding and outlet ducts (31,32) converge;
- a one-way interposition element (21) movable between a first position, where it allows a flow of inlet marked aerosol from said feeding duct (31) when breathing in, and a second position, where it stops the flow from said inhalation collector (10) into said feeding duct (31) of the outlet marked aerosol when breathing out,
**characterised in that** an element of inhalation (20), through nose or mouth, is provided mounted on said inhalation collector (10), and
wherein said one-way interposition element (21) is a flap valve, wherein said one-way interposition element (21) assumes intermediate positions between said first and second position during the breathing out cycle of the patient that reduces but does not stop the inlet marked aerosol flow into said inhalation collector (10).

2. Apparatus according to claim 1, wherein said flap valve comprises a stiff plastic foil that is elastically flexible.

3. Apparatus according to claim 2, wherein said flap valve deflects only one-way by an annular abutment (11) to which said foil is connected at a side and on which it rests when in closed position.

4. Apparatus according to claim 1, wherein said inhalation collector (10) is a three-way collector and has a central chamber with three apertures and precisely:
- a first opening for connecting said central chamber with a feeding duct (31) of marked aerosol (55) ;
- a second opening for connecting the central chamber with an outlet duct (32) of surplus marker connected to a radioactive material collecting filter;
- a third opening for connecting the central chamber with said element of inhalation (20) through nose or mouth;
- a first flap one-way valve at the first opening for an inlet flow;
- a second flap one-way valve at the second opening for an outlet flow.

5. Apparatus according to claim 1, wherein said inhalation collector (10) is a four-way collector and has a central chamber with four apertures and precisely:
- a first opening for connecting said central chamber with a feeding duct (31) of marked aerosol (55) ;
- a second opening for connecting the central chamber with an outlet duct (32) of surplus marker connected to a radioactive material collecting filter;
- a third opening for connecting the central chamber with a feeding duct (31) of air for non invasive mechanical ventilation;
- a fourth opening for connecting the central chamber with an element of inhalation (20) through nose or mouth;
- a flap one-way valve at the first opening for an inlet flow.

## Patentansprüche

1. Beatmungsgerät (1) für die pulmonale Szintigraphie, mit:
- einer Zuführleitung (31), die eine Einlassströmung eines markierten Aerosols (55) definiert,
- einer Auslassleitung (32) für ausgeatmetes, überschüssiges Auslassaerosol,
- einem Inhalationskollektor (10), an dem die zuführ- und Auslassleitungen (31,32) zusammenlaufen,
- einen Einweg-Zwischenfügeelement (21), das zwischen einer ersten Position, in der es eine Strömung des markierten Einlassaerosols von der Zuführleitung (31) beim Einatmen zulässt, und einer zweiten Position, in der es die Strömung von dem Inhalationskollektor (10) in die Zuführleitung (31) des markierten Auslassaerosols beim Ausatmen unterbricht, bewegbar ist,
**dadurch gekennzeichnet, dass** ein Element für die Inhalation (20) durch Nase oder Mund, an dem Inhalationskollektor (10) angebracht vorgesehen ist, und
wobei das Einweg-Zwischenfügeelement (21) ein Klappenventil ist, wobei das Einweg-Zwischenfügeelement (21) zwischenpositionen zwischen der ersten und der zweiten Position während des Ausatmungszyklus des Patienten einnimmt, was die Strömung des markierten Einlassaerosols in den Inhalationskollektor (10) verringert aber nicht unterbricht.

2. Gerät gemäß Anspruch 1, wobei das Klappenventil eine steife Kunststofffolie umfasst, die elastisch flexibel ist.

3. Gerät gemäß Anspruch 2, wobei das Klappenventil durch einen ringförmigen Anschlag (11), an dem die Folie an einer Seite angebracht ist und an dem sie in der geschlossenen Stellung anliegt, nur in einer Richtung auslenkt.

4. Gerät gemäß Anspruch 1, wobei der Inhalationskollektor (10) ein Dreiwege-Kollektor ist und eine zentrale Kammer mit drei Öffnungen und speziell folgendes aufweist:
- eine erste Öffnung zum Verbinden der zentralen Kammer mit einer Zuführleitung (31) des markierten Aerosols (55),
- eine zweite Öffnung zum Verbinden der zentralen Kammer mit einer Auslassleitung (32) für Überschussmarker, die mit einem Sammelfilter für radioaktives Material verbunden ist,
- eine dritte Öffnung zum Verbinden der zentralen Kammer mit dem Element für Inhalation (20) durch Nase oder Mund,
- ein erstes Einweg-Klappenventil an der ersten Öffnung für eine Einlassströmung,
- ein zweites Einweg-Klappenventil an der zweiten Öffnung für eine Auslassströmung.

5. Gerät gemäß Anspruch 1, wobei der Inhalationskollektor (10) ein Vierwege-Kollektor ist und eine zentrale Kammer mit vier Öffnungen und speziell folgendes aufweist:
eine erste Öffnung zum Verbinden der zentralen Kammer mit einer Zuführleitung (31) des markierten Aerosols (55),
- eine zweite Öffnung zum Verbinden der zentralen Kammer mit einer Auslassleitung (32) für Überschussmarker, die mit einem Sammelfilter für radioaktives Material verbunden ist,
- eine dritte Öffnung zum Verbinden der zentralen Kammer mit einer Zuführleitung (31) für Luft für eine nichtinvasive mechanische Ventilation,
- eine vierte Öffnung zum Verbinden der zentralen Kammer mit einem Element der Inhalation (20) durch Nase oder Mund,
- ein Einweg-Klappenventil an der ersten Öffnung für eine Einlassströmung.

## Revendications

1. Appareil de ventilation (1) pour la scintigraphie pulmonaire, comprenant :
un conduit d'alimentation (31) définissant un écoulement d'entrée de l'aérosol marqué (55) ;
un conduit de sortie (32) pour l'aérosol de sortie expiré / en surplus ;
un collecteur d'inhalation (10) où lesdits conduits d'alimentation et de sortie (31, 32) convergent ;
un élément d'interposition à une voie (21) mobile entre une première position, où il permet un écoulement de l'aérosol marqué d'entrée à partir dudit conduit d'alimentation (31) lorsque l'on respire dans celui-ci, et une seconde position, où il arrête l'écoulement provenant dudit collecteur d'inhalation (10) dans ledit conduit d'alimentation (31) de l'aérosol marqué de sortie lorsque l'on expire,
**caractérisé en ce que** l'on prévoit un élément d'inhalation (20), par le nez ou la bouche, qui est monté sur ledit collecteur d'inhalation (10), et
dans lequel ledit élément d'interposition à une voie (21) est une soupape à clapet, dans lequel ledit élément d'interposition à une voie (21) prend des positions intermédiaires entre lesdites première et seconde positions pendant le cycle d'expiration du patient qui réduit, mais qui n'arrête pas l'écoulement de l'aérosol marqué de sortie dans ledit collecteur d'inhalation (10).

2. Appareil selon la revendication 1, dans lequel ladite soupape à clapet comprend une feuille en plastique rigide qui est élastiquement flexible.

3. Appareil selon la revendication 2, dans lequel ladite soupape à clapet dévie uniquement une voie avec une butée annulaire (11) à laquelle ladite feuille est raccordée au niveau d'un côté et sur laquelle elle repose lorsqu'elle est dans la position fermée.

4. Appareil selon la revendication 1, dans lequel ledit collecteur d'inhalation (10) est un collecteur à trois voies et a une chambre centrale avec trois ouvertures et précisément :
une première ouverture pour raccorder ladite chambre centrale avec un conduit d'alimentation (31) de l'aérosol marqué (55) ;
une deuxième ouverture pour raccorder la chambre centrale avec un conduit de sortie (32) du marqueur de surplus raccordé à un filtre de collecte de matériau radioactif ;
une troisième ouverture pour raccorder la chambre centrale avec ledit élément d'inhalation (20) par le nez ou la bouche :
une première soupape à clapet à une voie au niveau de la première ouverture pour un écoulement d'entrée ;
une seconde soupape à clapet à une voie au niveau de la seconde ouverture pour un écoulement de sortie.

5. Appareil selon la revendication 1, dans lequel ledit collecteur d'inhalation (10) est un collecteur à quatre voies et a une chambre centrale avec quatre ouvertures et précisément :
une première ouverture pour raccorder ladite chambre centrale avec un conduit d'alimentation (31) de l'aérosol marqué (55) ;
une deuxième ouverture pour raccorder la chambre centrale avec un conduit de sortie (32) du marqueur de surplus raccordé à un filtre de collecte de matériau radioactif ;
une troisième ouverture pour raccorder la chambre centrale avec un conduit d'alimentation (31) d'air pour une ventilation mécanique non invasive ;
une quatrième ouverture pour raccorder la chambre centrale avec un élément d'inhalation (20) par le nez ou la bouche ;
une soupape à clapet à une voie au niveau de la première ouverture pour un écoulement d'entrée.
